# EUROPÄISCHE PATENTSCHRIFT

(11) **EP 0 982 294 B1**
(45) Veröffentlichungstag und Bekanntmachung des Hinweises auf die Patenterteilung: **23.01.2002**
(21) Anmeldenummer: 99115238.0
(22) Anmeldetag: 02.08.1999
(51) Int. Cl.: C07C 323/12, C07C 319/02, A23L 1/226, A61K 7/46

(54) **2-Mercapto-2-methyl-pentan-1-ol und seine Anwendung als Duft- oder Aromastoff**
2-Mercapto-2-methyl-pentan-1-ol and its use as perfuming or flavouring agent
2-Mercapto-2-méthyl-pentan-1-ol et son utilisation comme agent parfumant ou aromatisant

(30) Priorität: 20.08.1998 DE 19837703
(43) Veröffentlichungstag der Anmeldung: 01.03.2000
(73) Patentinhaber: Dragoco Gerberding & Co Aktiengesellschaft, 37603 Holzminden (DE)
(72) Erfinder: Sabater-Lüntzel, Christopher, Dr., 37671 Höxter-Ottbergen (DE); Widder, Sabine, Dr., 37603 Holzminden (DE); Pickenhagen, Wilhelm, Dr., 37671 Höxter (DE); Vössing, Tobias, 37688 Beverungen (DE)
(74) Vertreter: Eisenführ, Speiser & Partner

(56) Entgegenhaltungen:
- DE-A- 2 417 385
- DATABASE CHEMABS [Online] CHEMICAL ABSTRACTS SERVICE, COLUMBUS, OHIO, US STN, CAPLUS accession no. 1988:508851 , XP002120546 & A. OLSEN ET AL: CARLSBERG RES. COMMUN., Bd. 53, Nr. 1, 1988, Seiten 1-9,
- W. PICKENHAGEN ET AL: HELV. CHIM. ACTA, 1984, Seiten 947-952, XP002120545

## Beschreibung

Die Erfindung betrifft einen neuen Duft- und Aromastoff sowie ein Verfahren zur Herstellung dieses neuen Stoffes.

Lebensmittel werden in der heutigen Zeit regelmäßig aromatisiert. Die Mehrheit der Konsumenten in den modernen Industriegesellschaften erwartet nämlich eine breite Palette schmackhafter Lebensmittel zu erschwinglichen Preisen. Die Schmackhaftigkeit von Lebensmitteln ist von großer Bedeutung, da sie in der Regel eine gute Bekömmlichkeit bewirkt. Die Aromenindustrie stellt bereits eine Vielzahl von Aromen zur Verfügung, um Lebensmittel einer breiten Bevölkerungsschicht verfügbar und schmackhaft zu machen.

Aromastoffe werden eingesetzt, um (a) Lebensmittelprodukten ohne Eigengeschmack eine Geschmacksnote zu verleihen oder um (b) Aromaverluste auszugleichen, die beispielsweise im Rahmen des Herstellungsprozesses eines Lebensmittels auftreten.

Aus der DE-A- 2 417 385 sind bereits Mercapto-Alkanole der allgemeinen Formel

R₂₄-C(SH(R₂₅)-C(OH)(R₂₆)-R₂₇

bekannt, worin jede der Gruppe R₂₄ bis R₂₇, die gleich oder unterschiedlich sind, ein Wasserstoffatom oder eine niedrige Alkylgruppe mit 1 bis 3 Kohlenstoffatome und bevorzugt eine Methylgruppe bedeutet.

Als besonders bevorzugtes Mercapto-Alkanol wird 2-Mercapto-3-butanol angegeben.

Die genannten Mercapto-Alkanole können als Aromastoffe verwendet werden, ihr Schwellenwert kann im Bereich von 0,002 bis 0,2 ppm liegen.

Aus der DATABASE CHEMABS [Online] CHEMICAL ABSTRACTS, CAPLUS accession no. 1988:508851, Bd.53, Nr. 1, 1988, Seiten 1-9) ist die Verbindung 2-Mercapto-3-methyl-1-butanol bekannt, bei der es sich um einen Geschmacksstoff handeln soll.

Der vorliegenden Erfindung lag die Aufgabe zugrunde, einen neuen Stoff zur Aromatisierung von Lebensmitteln (nachfolgend auch kurz "Aromastoff" genannt) anzugeben.

Erfindungsgemäß wird die Verbindung 2-Mercapto-2-methyl-pentan-1-ol, deren Strukturformel nachstehend angegeben ist, als Aromastoff angegeben.

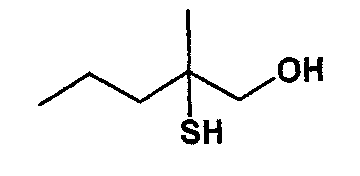

Überraschenderweise hat sich nämlich gezeigt, daß diese neu synthetisierte Verbindung hervorragend dazu geeignet ist, Lebensmitteln eine interessante geschmackliche und geruchliche Note zu verleihen. Auf Grund der sehr hohen geruchlichen und geschmacklichen Intensität der erfindungsgemäßen Verbindung kann sie in großer Verdünnung eingesetzt werden; die genaue Konzentration oder Menge zur Aromatisierung eines Lebensmittels wird der Fachmann auf übliche Weise an die jeweiligen Wünsche und Bedürfnisse des Einzelfalls anpassen.

Die erfindungsgemäße Verbindung ist überdies auch zur Verwendung als Duftstoff geeignet, und zwar insbesondere als Duftstoff in der Parfümindustrie; sie hat einen besonders niedrigen Geruchsschwellenwert, was sich als vorteilhaft erweist, da bereits geringe Mengen der erfindungsgemäßen Verbindung zur Erzeugung eines erwünschten Geruches ausreichen.

Die Erfindung betrifft neben der erfindungsgemäßen Verbindung selbst auch ein Verfahren zu ihrer Herstellung.

Bei diesem erfindungsgemäßen Verfahren zur Herstellung von 2-Mercapto-2-methyl-pentan-1-ol wird 2,3-Epithio-2-methyl-pentan-1-ol einer Reduktionsbehandlung unterzogen.

Entsprechend dem bevorzugten Einsatzgebiet der erfindungsgemäßen Verbindung betrifft die Erfindung auch Duft- oder Aromastofformulierungen, die die erfindungsgemäße Verbindung umfassen.

Und schließlich betrifft die Erfindung noch aromatisierte Lebensmittel mit einem Anteil an 2-Mercapto-2-methyl-pentan-1-ol sowie die Verwendung dieser Verbindung als Duft- oder Aromastoff.

Nachfolgend wird die Erfindung anhand von Beispielen näher erläutert:

### Beispiel 1: Herstellung von 2-Mercapto-2-methyl-pentan-1-ol

### 1.1. Herstellung von trans-2,3-Epoxy-2-methyl-pentan-1-ol durch Epoxidierung von trans-2-Methyl-2-penten-1-ol (CAS-Nr.[16958-19-3])

20g (0,2 mol) 2-Methyl-2-penten-1-ol (leicht erhältlich aus der Lithiumaluminiumhydridreduktion des kommerziell erhältlichen 2-Methyl-2-pentenals) werden in 200ml Methylenchlorid gelöst und im Eisbad auf 0°C abgekühlt. Man gibt nun in kleinen Portionen 59g (0,36 mol) feste m-Chlorperbenzoesäure (ca. 70%-ig) hinzu und läßt noch 2h bei 0°C und über Nacht bei Raumtemperatur weiterrühren. Anschließend gibt man 8g Calciumhydroxid hinzu und läßt 1h rühren. Es wird abfiltriert, und der Filterrückstand wird gut mit Diethylether nachgewaschen. Die vereinigten organischen Phasen werden jeweils einmal mit 50 ml 5%iger Natriumcarbonatlösung und 50 ml gesättigter Kochsalzlösung gewaschen, über Natriumsulfat getrocknet, filtriert und das Lösungsmittel vorsichtig im Teilvakuum abgezogen. Ausbeute 65%. Der resultierende Epoxyalkohol wird ohne weitere Reinigung in die nächste Reaktion eingesetzt.
Ausbeute 15,08 g (65%).

### trans-2,3-Epoxy-2-methyl-pentan-1-ol

**MS** (EI, 70 eV): kein M⁺, 74 (15), 59 (60), 58 (99), 57 (58), 43 (100), 41 (29), 39 (21)

### 1.2. Herstellung von 2,3-Epithio-2-methyl-pentan-1-ol aus trans-2,3-Epoxy-2-methyl-pentan-1-ol

In eine trockene 250ml-Rührapparatur gibt man unter Stickstoffatmosphäre 1,57g (20,6 mmol) Thioharnstoff, 100ml Tetrahydrofuran und 2g (17 mmol) des gemäß 1.1. dargestellten Epoxyalkohols trans-2,3-Epoxy-2-methyl-pentan-1-ol. Bei Raumtemperatur tropft man langsam 5,87g (20,6 mmol) Titantetraisopropylat hinzu. Nachdem sich der Thioharnstoff gelöst hat, läßt man noch 2h bei Raumtemperatur nachrühren. Man gibt nun 70ml Diethylether und 35ml gesättigte Natriumhydrogencarbonatlösung hinzu und läßt 1h weiterrühren. Die Suspension wird abgenutscht, und der Filterkuchen wird dreimal mit je 30ml Diethylether und dann mit zweimal 30 ml Methylenchlorid gewaschen. Die vereinigten organischen Phasen werden zweimal mit je 70ml Wasser und einmal mit 70 ml gesättigter Natriumchloridlösung gewaschen, über Natriumsulfat getrocknet, filtriert und das Lösungsmittel vorsichtig im Teilvakuum abgezogen. Das Produkt wird ohne weitere Reinigung in die nächste Reaktion eingesetzt.
Ausbeute: 1,08 g (75%).

### 2,3-Epithio-2-methyl-pentan-1-ol

**MS** (EI, 70 eV): 132 (M⁺ 79), 101 (12), 99 (59), 98 (10), 85 (14), 81 (17), 75 (20), 74 (50), 73 (15), 71 (33), 70 (22), 69 (21), 67 (19), 61 (20), 59 (100), 58 (31), 57 (25), 55 (23), 53 (14), 47 (14), 45 (28), 43 (54), 41 (71), 39 (36)

### 1.3. Reduktive Öffnung des Thiirans 2,3-Epithio-2-methyl-pentan-1-ol zum 2-Mercapto-2-methyl-pentan-1-ol

Zu einer Lösung aus 6,22g Natrium-bis-(2-methoxyethoxy)-aluminiumdihydrid (65 Gew.-% in Toluol, 30,8 mmol) und 30ml Diethylether wird unter Stickstoffatmosphäre bei -20°C langsam eine Lösung des gemäß 1.2. dargestellten Thiirans (3,87 g, 29,3 mmol) in 30ml Diethylether getropft. Nach dem Zutropfen läßt man noch 5h bei Raumtemperatur nachrühren. Bei 0°C hydrolysiert man sehr vorsichtig zuerst mit 5ml Wasser und anschließend mit 10%-iger Schwefelsäure. Die Phasen werden getrennt, die wäßrige wird einmal mit 20ml Diethylether extrahiert. Die vereinigten organischen Phasen werden mit 30 ml gesättigter Natriumhydrogencarbonatlösung gewaschen, über Natriumsulfat getrocknet, filtriert und das Lösungsmittel vorsichtig im Teilvakuum abgezogen. Man erhält ein Rohprodukt, welches säulenchromatographisch (79% Hexan/14% Methylenchlorid/7% Diethylether) aufgereinigt wird.
Ausbeute: 380mg (9,7%)

Spektroskopische Daten von 2-Mercapto-2-methyl-pentan-1-ol:
**IR** (Gasphase): 3668 (w), 3577 (w), 2968 (s), 2584 (w), 2941 (s), 2884 (m), 1463 (m), 1386 (m), 1043 (m)
w = schwache, m = mittlere, s = starke Bande
^{**1**}**H-NMR** (300 MHz, d₆-Benzol, 300 K, interner Standard: TMS): δ = 0.8 (triplettartiges Multiplett, 3H, -CH₃), 1.1 (s, 3H,-CH₃), 1.2-1.4 (m, 5H, 2mal CH₂ und SH), 2.0 (brs, 1 H, OH), 3.2 (brs, 2H, CH₂)
Multiplizitäten: s = Singulett, brs = breites Singulett, m = Multiplett
^{**13**}**C-NMR** (75 MHz, d₆-Benzol, 300 K, interner Standard: TMS): δ = 72.2, 50.1, 42.9, 25.9, 18.0, 14.6
**MS** (EI, 70eV): 134 (M⁺, 10), 116 (0,1), 103 (100), 102 (80), 91 (8), 83 (25), 73 (18), 69 (74), 61 (95), 59 (40), 55 (38), 45 (36), 43 (20), 41 (60)

Diesen spektroskopischen Daten entsprechen die beigefügten Spektren von 2-Mercapto-2-methyl-pentan-1-ol. Es stellen dar:

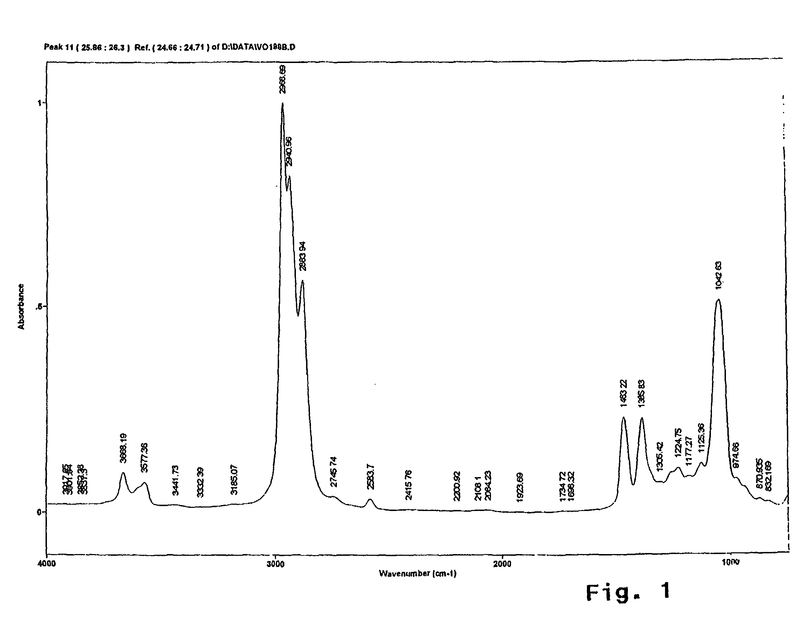

- Fig. 1: IR-Spektrum

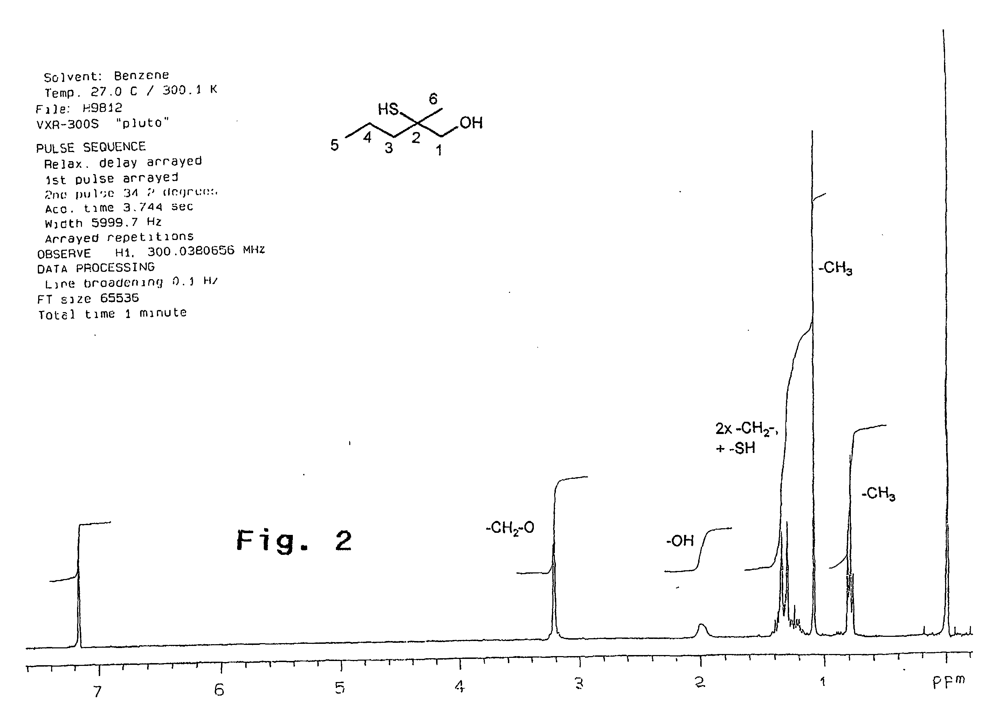

- Fig. 2: ¹H-NMR-Spektrum

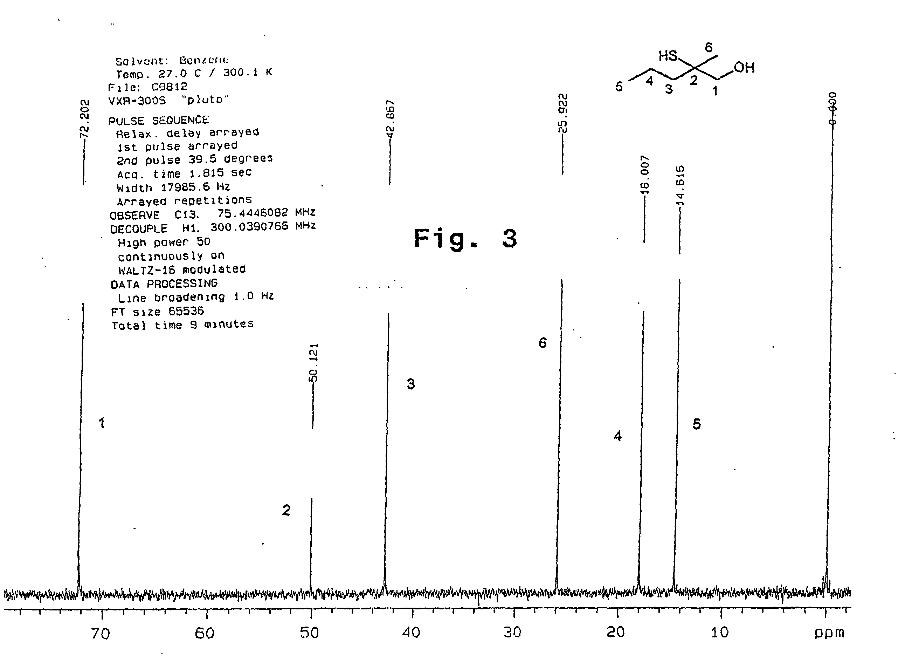

- Fig. 3: ¹³C-NMR-Spektrum

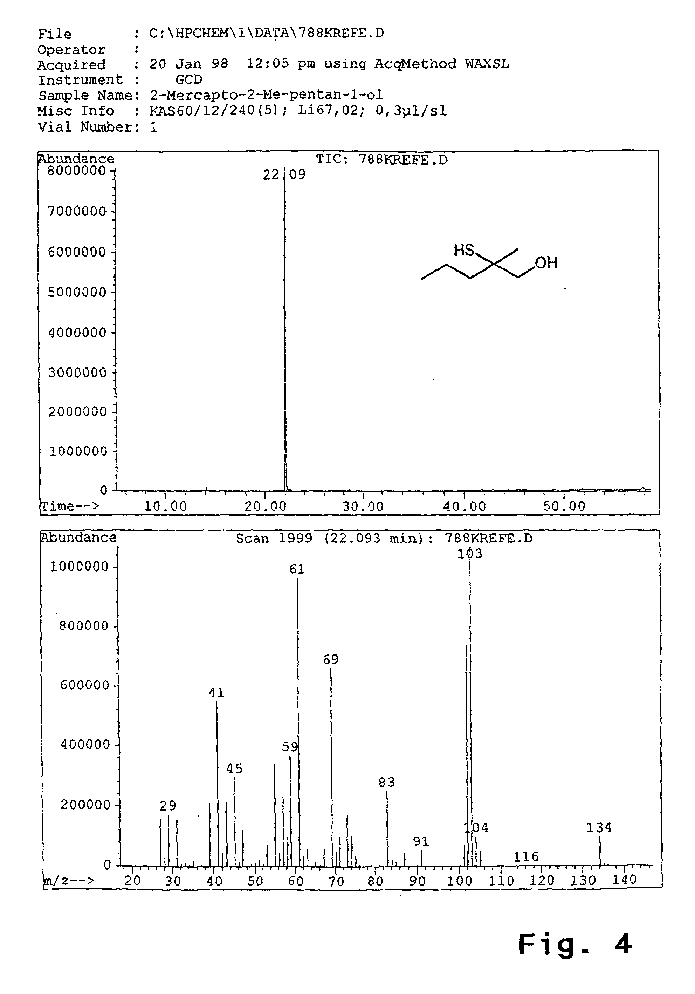

- Fig. 4: Massenspektrum

### Beispiel 2: Sensorische Untersuchung des 2-Mercapto-2-methyl-pentan-1-ol

Geruchsbeschreibung:
verbranntes Plastik, Gummi, stechend, Grapefruit, Cassis, stark

Geschmacksbeschreibung:
schwefelig, aldehydisch, tropische Früchte, stechend, Gummi, Grapefruit

Geruchsschwellenwert:
0,1 ppb (0,1 µg/l Wasser)

## Patentansprüche

1. 2-Mercapto-2-methyl-pentan-1-ol, insbesondere zur Verwendung als Duft- oder Aromastoff.

2. Verfahren zur Herstellung von 2-Mercapto-2-methyl-pentan-1-ol, wobei 2,3-Epithio-2-methyl-pentan-1-ol reduziert wird.

3. Verwendung von 2-Mercapto-2-methyl-pentan-1-ol als Duft- oder Aromastoff.

4. Aromatisiertes Lebensmittel mit einem Anteil an 2-Mercapto-2-methylpentan-1-ol.

## Claims

1. 2-Mercapto-2-methyl-pentan-1-ol, in particular for use as perfuming or flavouring agent.

2. Method for the preparation of 2-mercapto-2-methylpentan-1-ol, in which 2,3-epithio-2-methyl-pentan-1-ol is reduced.

3. Use of 2-mercapto-2-methyl-pentan-1-ol as perfuming or flavouring agent.

4. Flavoured food with a 2-mercapto-2-methyl-pentan-1-ol content.

## Revendications

1. 2-mercapto-2-méthyl-pentan-1-ol, notamment pour une utilisation comme agent parfumant ou aromatisant.

2. Procédé destiné à la fabrication de 2-mercapto-2-méthyl-pentan-1-ol, moyennant quoi le 2,3-épithio-2-méthyl-pentan-1-ol est réduit.

3. Utilisation de 2-mercapto-2-méthyl-pentan-1-ol comme agent parfumant ou aromatisant.

4. Produit alimentaire aromatisé avec une fraction de 2-mercapto-2-méthyl-pentan-1-ol.
